# EUROPEAN PATENT APPLICATION

(11) **EP 0 955 367 A2**
(43) Date of publication of application: **10.11.1999**
(21) Application number: 99107630.8
(22) Date of filing: 16.04.1999
(51) Int. Cl.: C12N 15/40, C07K 14/175, C12Q 1/68, G01N 33/68

(54) **Recombinant nucleoprotein of toscana virus**

(30) Priority: 24.04.1998 IT MI980889
(71) Applicant: DIESSE DIAGNOSTICA SENESE S.p.A., I-20123 Milano (IT)
(72) Inventor: Soldateschi, Dario, 53100 Siena (IT); Cusi, Maria Grazia, 53100 Siena (IT); Valassina, Marcello, 58100 Grosseto (IT)
(74) Representative: Mancini, Vincenzo, Dr.

(57) **Abstract**

A recombinant nucleoprotein of Toscana virus, a process for its preparation and purification and a method for the detection of antibodies directed to Toscana virus are described.

SEQUENCE LISTING

<110> DIESSE DIAGNOSTICA SENESE S.R.L.

<120> RECOMBINANT NUCLEOPROTEIN OF TOSCANA VIRUS

<130> g67703VM/mg

<140>
<141>

<150> MI98A000889
<151> 1998-04-24

<160> 5

<170> PatentIn Ver. 2.1

<210> 1
<211> 253
<212> PRT
<213> Toscana virus

<400> 1
Met Ser Asp Glu Asn Tyr Arg Asp Ile Ala Leu Ala Phe Leu Asp Glu
  1               5                  10                  15

Ser Ala Asp Ser Gly Thr Ile Asn Ala Trp Val Asn Glu Phe Ala Tyr
             20                  25                  30

Gln Gly Phe Asp Pro Lys Arg Ile Val Gln Leu Val Lys Glu Arg Gly
         35                  40                  45

Thr Ala Lys Gly Arg Asp Trp Lys Lys Asp Val Lys Met Met Ile Val
     50                  55                  60

Leu Asn Leu Val Arg Gly Asn Lys Pro Glu Ala Met Met Lys Lys Met
 65                  70                  75                  80

Ser Glu Lys Gly Ala Ser Ile Val Ala Asn Leu Ile Ser Val Tyr Gln
                 85                  90                  95

Leu Lys Glu Gly Asn Pro Gly Arg Asp Thr Ile Thr Leu Ser Arg Val
            100                 105                 110

Ser Ala Ala Phe Val Pro Trp Thr Val Gln Ala Leu Arg Val Leu Ser
        115                 120                 125

Glu Ser Leu Pro Val Ser Gly Thr Thr Met Asp Ala Ile Ala Gly Val
    130                 135                 140

Thr Tyr Pro Arg Ala Met Met His Pro Ser Phe Ala Gly Ile Ile Asp
145                 150                 155                 160

Leu Asp Leu Pro Asn Gly Ala Gly Ala Thr Ile Ala Asp Ala His Gly
                165                 170                 175

Leu Phe Met Ile Glu Phe Ser Lys Thr Ile Asn Pro Ser Leu Arg Thr
            180                 185                 190

Lys Gln Ala Asn Glu Val Ala Ala Thr Phe Glu Lys Pro Asn Met Ala
        195                 200                 205

Ala Met Ser Gly Arg Phe Phe Thr Arg Glu Asp Lys Lys Lys Leu Leu
    210                 215                 220

Ile Ala Val Gly Ile Ile Asp Glu Asp Leu Val Leu Ala Ser Ala Val
225                 230                 235                 240

Val Arg Ser Ala Glu Lys Tyr Arg Ala Lys Val Gly Lys
                245                 250

<210> 2
<211> 25
<212> PRT
<213> Toscana virus

<400> 2
Met Gly Ser Ser His His His His His His Ser Ser Gly Leu Val Pro
  1               5                  10                  15

Arg Gly Ser His Met Leu Glu Asp Pro
             20                  25

<210> 3
<211> 1869
<212> DNA
<213> Toscana virus

<400> 3
acacaaagac ctcccgtatt gctaaaccag agctagtaat agacttctag acagccatgc 60
aatccagagc tgtcatcttg aagtatagat ctggttcagg ccacaagagg tctttgccca 120
ggttctacat agactgtgat ttggacacct ttgattttga gaaggattgc tctctgattg 180
agaatgagtt ccccatttac ataaacaatt ataaggtggt ctataagtca aagccaactc 240

tctcacattt cctcattgag aaggagtttc ctgctgtgct ggggcctggt atgatcagtg 300
cagttcgaac cagactttac gagccaacta tgagagagct ctaccaggaa tcgattcacc 360
aactaaagag gagcaacaag aaataccttt tgtctgctct caggtggccc acagggattc 420
ctactctaga gtttatagac tattacttcg aggagctcct gttcttgtca gagtttgacc 480
cggggtctat ccagagatac ctgaaattac tggttaaggc ctctgggctt tacaactcca 540
ctaatgagga gcagatagtg gagattcaca gacgagtgct catagaaggc aaaaagcacg 600
gattgactgc ttttgatctc ccaggaaatg acatccttgg agacatctgt gtggtccaag 660
cagcacgggt gacaagactg gttgctaaga cattctctaa gatgaccaga gacacccatc 720
tgatgatata cttctcgata agcccagttg agttggtttt gagtaaactt gataagaaag 780
gggacaagag ggctaaagca aaagggttga tgtctatgag tgccgctagg tcttatgact 840
attttatgag aactgacttg ggattcagag agactgctct ttccaccttt tgggctaagg 900
actggcctac cccacaagag accattctat ctgacaaacg atgccttaaa gaagacatga 960
gagtgacaaa gtggctgcct agtccccccc actacccacc cttatgatcc cacccctcag 1020
atttccccaa tccccatccc aatcctaatt cccctaaccc cctggcagct cacttgccaa 1080
ccttggcgcg atacttctca gcactcctca ctactgcaga cgccagaaca agatcctcat 1140
caatgatccc cactgctatg agcagcttct tcttatcttc tctggtgaag aatcgtccac 1200
tcatggcggc catattgggc ttttcaaatg tggcagccac ttcattagcc tgttttgttc 1260
tcagagatgg atttatggtc tttgagaact caatcatgaa cagcccatga gcatcagcaa 1320
tagtggctcc agctccattc ggaaggtcca gatcaatgat cccagcaaag ctagggtgca 1380
tcatggctct tgggtaggtt acaccagcaa tggcatccat agtggtccca gacacgggca 1440
gggattctga caggacacgt agtgcctgaa cagtccacgg aacaaatgcg gctgacaccc 1500
ttgacagagt gatggtgtcc ctgccagggt tcccttcttt caactggtag actgaaatca 1560
ggttggccac aatagaagca cccttctctg acatcttctt catcatggcc tctggcttgt 1620
ttcccctgac aaggttcagc acaatcatca tcttcacatc tttcttccag tccctgccct 1680
ttgccgttcc tctctccttg accaactgaa caatgcgctt tggatcaaac ccttggtaag 1740
caaactcatt cacccatgca ttgatggtcc cagaatctgc agactcatca agaaaagcaa 1800
gagcaatatc gcgataattc tcgtctgaca tgttgatagc ttttgtttaa tacacgggaa 1860
tctctgtgt 1869

<210> 4
<211> 24
<212> DNA
<213> Artificial Sequence

<220>
<223> Description of Artificial Sequence: primer_bind

<400> 4
tcggatccca tgtcagacga gaat 24

<210> 5
<211> 23
<212> DNA
<213> Artificial Sequence

<220>
<223> Description of Artificial Sequence: primer_bind

<400> 5
atggatcctc acttgccaac ctt 23

## Description

The present invention relates to a recombinant protein of Toscana virus.

In particular, the present invention relates to a recombinant nucleoprotein of Toscana virus, a process for its preparation and purification and a method for the detection of antibodies directed to Toscana virus.

Toscana virus (TV) belongs to the Bunyaviridae family, Phlebovirus genus. The genome consists of 3 Circular segments of negative single-stranded RNA (Accardi et al., Virus Res. 27 119-131, 1993, Schmalijohn, C.S. in B.N. Fields, D.M. Knipe, P.M. Howley (ed) VIROLOGY, Lippincott-Raven, Philadelphia-New York 1995, Gro', M.C. et al., Virology 191: 435-438, 1992).

The large segment (L) codes for the viral polymerase, the medium segment (M) for the two glycoproteins of G1 and G2 pericapside and the small segment (S) for the nucleoprotein (N) and the non-structural protein (NS).

TV is endemic in Italy (Nicoletti, L. ed al., Am. J. Trop. Med. Hyg. 45: 429-434; 1991, Braito, A. et al., Eur. J. Epidemiol. 13: 761-764 1997) and Cyprus (Eitrem, R. et al., Epidemiol. Infect. 107: 685-691, 1991) and, as it is demonstrated by cases of infection in tourists returning from such countries, in Portugal (Ehrnst, A. et al., The Lancet: i: 1212-1213, 1985) and Spain (Eitrem et al., Research in Virology 142: 387-394).

Cases of infection with TV in tourists returning from endemic countries have been described in the United States (Calisher, C.H. et al., The Lancet i: 165-166, 1987), Sweden (Eitrem. R. et al. Scand. J. Infect. Dis. 23, 451-457, 1991) and Germany (Schwarz, T.F. et al., The Lancet: 342 803-804, 1993). TV is transmitted by insects of Phlebotomus genus. In Italy it was isolated from Phlebotomus perniciosus (Verani et al., Acta Virol. 28: 39-47, 1984).

The disease caused by TV, described for the first time in Herzegovina (Pick, A., Wiener Medizinische Wochenschrift 36: 1141-1145, 1886), is characterized by fever, intense headache and aseptic meningitis (Nicoletti L. et al., Am. J. Trop. Med. Hyg, 45 429-434, 1991).

A diagnosis of infection with TV is performed on the serum, by detecting the presence of specific antibodies of the IgG and IgM classes, by means of indirect immuno fluorescence, immuno enzymatic methods or serum neutralization of virus (Eitrem, R. et al., Am. J. Trop. Med. Hyg 43: 207-211, 1990; Schwarz, T.F. et al., Epidemiol Infect. 114, 501-510, 1995). The technique of the polymerase chain reaction (PCR) for the amplification of genomic sequences from serum or cephalorachidian liquid was recently used for diagnostic purposes. (Schwarz, T.F. et al., Research in Virology 146 355-362, 1996; Valassina, M. et al., J. Clin. Microbiol. 34: 2500-2502, 1996).

Such methods suffer from some intrinsic limitations. Indirect immuno fluorescence can present a subjective evaluation and poor sensitivity for detecting IgMs, while serum neutralization of virus is a long laborious process. Furthermore, these methods, together with the immuno enzymatic methods hitherto employed, are not standardized and require a manipulation of virus and thus they need equipment and safety levels that are not easily available in common serology laboratories.

On the other hand, the polymerase chain reaction is a highly sensitive method, nevertheless it requires skilled staff and it is not yet a routine activity of most laboratories.

Lately, a study performed with the Western blotting method upon three sera from patients infected with TV pointed out a prevalent antibody reaction against the nucleoprotein of virus (Schwarts, T. F. et al., J. Med. Virol. 49, 83-86, 1996).

This protein, obtained by using the recombinant DNA technology, could thus be a good substitute of the virus in the diagnostic field, having the advantages of being non-infectious and easy to standardize.

An object of the present invention is a recombinant nucleoprotein of Toscana virus having the SEQ ID No: 1 or including the SEQ ID No: 1, or a portion or a modification thereof, that is reactive with antibodies from subjects infected with TV, where the infection is under way or previous, said SEQ ID No: 1 or portion or modification being optionally preceded and/or followed by amino acid sequences that do not alter the antigen properties and the biological functions of the nucleoprotein and/or that are required for the expression, isolation and/or purification of SEQ ID No: 1 or portion or modification.

In particular, a nucleoprotein having the SEQ ID No: 1 or one in which the SEQ ID No: 1 is immediately preceded by the SEQ ID No: 2 are preferred.

In the present invention, the expression "recombinant protein of Toscana virus" means the whole nucleoprotein whose molecular weight is 28KDa, having the SEQ ID No: 1 consisting of 253 amino acids (aa) or such proteins containing the SEQ ID No: 1 or a portion thereof that is reactive with antibodies of subjects infected with TV, where the infection is under way or previous, optionally preceded and/or followed by amino acid sequences that do not alter the antigen properties and the biological functions of the nucleoprotein of the invention and/or that are required for the expression, isolation and/or purification of the SEQ ID No: 1, such as, for example, a protein consisting of the SEQ ID No: 1 preceded by the SEQ ID No: 2, produced in a suitable expression system. Suitable expression systems to accomplish the present invention are prokaryotes and eukaryotes such as, for example, bacteria (e.g. Escherichia coli and Bacillus subtilis), yeasts, for example Saccharomyces cerevisiae, mammal cells (for example CHO cells) and insect cells (for example SF9 Cells).

The possibility to perform small alterations in the amino acids of the sequence of nucleoprotein of the invention without destroying its biological functions and obtaining thus proteins having antigenic properties equivalent to those of the nucleoprotein having the SEQ ID No: 1, will also be apparent to those skilled in the art.

The aa's 1-25 composing the SEQ ID No: 2 result from the expression vector pET-15b (manufactured by Novagen, WI, USA) that is used for the production of the nucleoprotein of the invention. The sequence having six molecules of hhystidine, instead used for purificatio, is in position5-10, while the aa's in position 14-19 are the sequence that is recognized by the thrombine proteolytic enzyme, wich can be used if it is desired to remove most aa's extraneous to the SEQ ID No: 1.

The S segment of viral RNA coding for the NS protein and the recombinant nucleoprotein of the invention has the SEQ ID No: 3, expressed as cDNA.

The sequence of RNA coding for the nucleoprotein of TV, that does not usually include introns, corresponds to nucleotides 1070-1831 and encodes from right to left.

A further object of the present invention is a process for producing and purifying the recombinant nucleoprotein of Toscana virus.

In particular, a further object of the invention is a process for producing recombinant nucleoprotein of Toscana virus, comprising:
- transcription of RNA corresponding to nucleotides 1070-1831 of the S segment of TV RNA in cDNA, through reverse transcriptase;
- amplification of the resulting cDNA, by means of the polymerase chain reaction (PCR), using specific primers containing at the 5' end the nucleotide sequence recognized by an appropriate restriction enzyme;
- digestion of amplified cDNA by said enzyme which is then embedded into a suitable expression plasmid in the cloning site of said enzyme;
- transformation with the said recombinant plasmid in a suitable expression system, either a prokaryote or a eukaryote, such as, for example, bacteria, e.g. Escherichia coli and Bacillus subtilis, yeasts, for example Saccharomyces cerevisiae, mammal cells, e.g. CHO cells, and insect cells, e.g. SF9 Cells.
- expression of said nucleoprotein in said expression system;
- recovery, purification of the recombinant nucleoprotein and possibly removal of amino acids extraneous to the SEQ ID No: 1 or a portion or a modification thereof, that is reactive with antibodies from subjects infected with TV, where the infection is under way or previous, said SEQ ID No: 1 or portion or modification being optionally preceded and/or followed by amino acid sequences that do not alter the antigen properties or the biological functions of the nucleo protein and/or that are required for the expression, isolation and/or purification of said SEQ ID No: 1 or portion or modification.

The recombinant protein of the invention is preferably produced through expression in Escherichia coli of cDNA of the gene encoding for nucleoprotein, located in the S segment of viral RNA sequenced by Giorgi C. et al., Virology 180: 738-753, 1991, included by reference in the present description.

In particular, the process for producing and purifying the nucleo protein having the SEQ ID No: 1, possibly immediately preceded by SEQ ID No: 2, in which the production of said nucleo protein takes place by expression in Escherichia coli of cDNA of the gene coding for nucleoprotein, located in the S segment of viral RNA, comprises:
- transcription of RNA corresponding to nucleotides 1070-1831 of the S segment of TV RNA in cDNA, through reverse trascriptase;
- amplification of the resulting cDNA, through polymerase chain reaction (PCR), using specific primers including at 5' end the nucleotide sequence recognized by BamHI restriction enzyme;
- digestion of amplified cDNA by said enzyme and introduction into an expression plasmid, preferably the plasmid pET-15b (manufactured by Novagen, WI, USA), into the BamHI cloning site in frame with a sequence having 25aas encoded by pET-15b vector starting from start ATG under the control of the promoter recognized by T7 polymerase, and containing six consecutive molecules of hystidine;
- transformation with said recombinant plasmid in a suitable expression system, preferably in a strain of E. coli, in particular E. coli strain BL21 (ADE3);
- induction of expression of said nucleoprotein through addition to said bacterial culture of a suitable inducer, in case of pET-15b vector isopropylthio-β-galactoside (IPTG) or, in case of other expression vectors with different promoters, for example, indole-3-acrylic acid or nalidixic acid;
- purification of recombinant nucleoprotein through immobilized metal affinity chromatography (IMAC) and possible removal of amino acids of the SEQ ID No: 2 by digestion with thrombine.

Specific primers, suitable to perform the present invention through expression by pET-15b vector, are the following:
- Sense primer, having the SEQ ID No: 4; and
- Antisense primer, having the SEQ ID No: 5.

In the present invention, in frame cloning of a protein means the insertion of the sequence coding for the VT viral nucleoprotein, so that it is encoded as a fusion protein with the sequence having 25 aa's encoded by the pET-15b vector starting from start ATG under control of the promoter recognized by T7 polymerase.

The purification by immobilized metal affinity chromatography (IMAC) comprises the attack and subsequent specific eluition from the resin of recombinant nucleoprotein so as to separate it from most bacterial proteins.

In particular, the purification of nucleoprotein of the invention comprises:
- its extraction from bacteria by sonication;
- chromatography of supernatant on a chromatography column containing a resin with immobilized metal, preferably nickel, such as, for example, Ni-NTA resin (Qiagen, CA, USA).

It has been noticed that the recombinant nucleoprotein of TV can be obtained, according to the process of the invention, in a greater quantity and better purity than those obtainable by the traditional method of infection of cellular cultures.

The recombinant nucleoprotein obtained according to the process of the invention turns also out to be substantially purified, that is practically free of other proteins and/or molecules normally associated to it or that are present together with the protein in its natural environment.

The nucleoprotein of the invention also has such characteristics of purity and antigenicity that it can be used, according to methods known in the art, for the detection of Toscana virus.

As a matter of fact, a further object of the present invention is a method for the detection of antibodies directed to Toscana virus, comprising the use of the above illustrated recombinant nucleoprotein.

In particular, a method of enzyme immuno assay (EIA) for the detection of antibodies of the IgG and IgM classes is preferred, in which the recombinant protein of the invention is used in the place of the virus produced in cellular cultures or infected animals, to diagnose under way or previous infection.

In its application in the method for the detection of specific IgGs and IgMs, TV nucleoprotein is immobilized on solid phase to bind to specific antibodies present in the sample under investigation (serum). These are then detected through addition of anti-human IgGs or anti-IgMs monoclonal antibody or polyclonal antibodies that are labeled with peroxidase.

To detect specific Igms, in alternative to the method just described, in wich the nucleoprotein is immobilized, it is preferred to immobilize on the solid phase anti-human IgMs monoclonal antibody, or polyclonal antibodies, capturing the IgMs. The presence of IgMs specific for TV is then detected through addition of a biotinilated recombinant nucleoprotein-avidine complex labeled with peroxidase.

A further object of the invention consists in a diagnostic Kit comprising recombinant protein of Toscana virus.

The following examples illustrate the invention without limiting it.

### EXAMPLE 1

### Expression of TV nucleoprotein in E. coli.

Viral RNA is extracted from the virus as follows:
200 µl of a viral suspension comprising 2x10 plate forming units of TV are placed under stirring with 600 myl of extraction buffer (4 M guanidine thiocyanate, 25 mM sodium citrate, pH 7.0, sarkosil 2% (sodium salt of N-laurylsarcosine), 0.1 M β-mercaptoethanol, 2 myg tRNA). 50 myl of 2M sodium acetate -pH 4.0- 400 myl of phenol saturated with water and 100 myl of a mixture containing chloroform and isoamilic alcohol in the ratio of 49 to 1 are then added. The mixture is stirred and placed under ice. After 10 minutes the mixture is centrifugated at 10.000 xg for 15 minutes and the water phase is extracted. RNA is precipitated by addition of 2.5 volumes ethanol. The sediment, containing RNA, is washed with 70% ethanol and then suspendend again in 5 myl of sterile water treated with diethylpyrocarbonate.

RNA corresponding to nucleotides 1070-1831 of S segment of TV RNA is transcripetd in DNA by reverse transcription, as follows:
to 1 myg of RNA obtained as described above and denaturated for 5 minutes at 80°C 5 myl of desoxynucleotide triphosphates, 1.25 mM each, 5 myl of reverse transcriptase buffer (Tris-HCl 250 mM -pH 8.3-375 mM KCl, 15 mM MgCl, 50 mM dithiothreitol), 1 myl of sense primer as previously descripted (100ng), 0.5 myl (100-200 U) of reverse transcriptase, 0.5 myl of Rnase inhibitor are added, followed by sterile water to make up to 25 myl. The reaction mixture is then incubated for 30 minutes at 37°C. The sample thus reverse transcripted is amplified through polymerase chain reaction (PCR) using sense primers SEQ ID No: 4 and antisense primers SEQ ID No: 5 and TAQ thermo resistant polymerase with 40 Cycles under the following conditions: 1 minute at 94°C; 50 seconds at 56°C; 1 minute at 72°C.

Amplified DNA is digested with BamHI restriction enzyme and introduced into the expression plasmid pET-15b (Novagen, WI. USA), previously digested with the same enzyme.

This vector contains, in the 5'-3' direction (N-terminal - C terminal), the promoter recognized by polymerase of T7 phage followed by Lac operator and translation start ATG. After a short sequence having 4 amino acids (aa's) there is a sequence coding for six hystidines, a sequence coding for a sequence recognized by thrombine proteolytic enzyme and a BamHI cloning site. The vector also includes the gene for β-lactamase that confers ampicillin resistance.

The protein, whose sequence is cloned in frame in this vector, is expressed as a fusion protein with an amino acid sequence of 25 aa's including 6 consecutive molecules of hystidine (poly-hys) that is useful to promote purification through immobilized metal affinity chromatography (IMAC). After purification, aa's extraneous to the cloned sequence can be removed by digestion with thrombine.

The pET-15b vector containing the sequence for TV nucleoprotein is inserted in the JM 109 strain of E. coli (cloning host) as follows:
0.2 ml of competent bacterial cells and 5 myl of recombinant plasmid (100-200 ng) are placed in a sterile polypropylene tube. The sample is incubated under ice for 30 minutes, subjected then to a change in temperature of 90 seconds at 42°C and subsequently incubated again for 2 minutes under ice. 0.8 ml of SOC medium ( tripton 2%, yeast extract 0.5%, 10 mM NaCl, 2.5 mM KCl, 10 mM MgSO, 10 mM MgCl, 20 mM glucose) are then added and bacteria are incubated for 1 hour at 37°C under stirring. Successively, 0.1 ml of bacterial culture are plated on LB-Agar dishes (peptone 1%, yeast extract 0.5%, NaCl 0.5%, Agar 1.2%) containing 100 myg/ml of ampicillin, the plate is then incubated for 15 hours at 37°C. Some bacterial colonies are then collected and propagated in liquid medium containing 100 myg/ml of ampicillin. From these, plasmidic DNA is extracted and analyzed by restriction analysis as follows: plasmid is digested with BamHI restriction enzyme to test for the presence of the insert (DNA coding for the TV nucleoprotein); as a matter of fact, in plasmids in which this is present, following digestion with BamHI, electrophoretic analysis of DNA will show two bands of 855 bases (insert) and 5708 bases (vector). Furthermore, in order to test for the correct orientation of insert, plasmidic DNA is digested with NruI restriction enzyme; as a matter of fact, two fragments of 2022 and 4457 bases in case of correct orientation and of 2745 and 3734 bases in case of reverse orientation are obtained with such enzyme. Based on this analysis, some supposdly appropriate plasmids are chosen and inserted, by the method described above, into the BL21 (A DE3) strain of E. coli (expression host). Colonies isolated from this second transformation are cultured in liquid medium and induced by IPTG to test for the production of recombinant nucleoprotein. After incubating for three hours at 37°C, the total bacterial proteins are analyzed by polyacrylamide gel electrophoresis in the presence of sodium dodecylsulphate. Clones having a huge band of 28Kda PM, lacking in control bacteria not trasformed with plasmid, and that is recognized in Western blotting by positive sera for IgG anti TV, are thought to be suitable. Starting from one of these clones, a stock of ampoules with frozen bacteria is prepared, that will then serve for reproducing recombinant nucleoprotein on a large scale.

### EXAMPLE 2

### Purification of recombinant nucleoprotein

Recombinant nucleoprotein of TV is extracted from bacteria through sonication. After centrifugation at 10'000 xg, the supernatant is passed over a chromatographic column containing a resin with nickel (Ni-NTA, Qiagen, CA,USA), equilibrated in 50 mM phosphate buffer pH 8.0 Containing 300 mM NaCl. After passing the sample, the resin is washed with 50 mM phosphate buffer - pH. 6.0- containing 1 M NaCl and 10% glycerol. The recombinant nucleoprotein is eluted with a 0-0.5 M imidazole gradient. The thus purified protein is adapted, after dialysis against a suitable buffer, for being employed in an immunoenzymatc test for the detection of anti TV antibodies.

### EXAMPLE 3

### ELISA with recombinant nucleoprotein of the invention.

Recombinant nucleo protein of Toscana virus is immobilized on the solid phase of micro plate wells for immuno enzymatic assays through incubation in carbonate buffer at room temperature. The wells are then washed four times with washing buffer [PBS (10 mM sodium phosphate, 150 mM sodium chloride, pH 7.4) + Brij 35 (polyoxyethylene lauryl ether 0.05%)], 100 myl of assay human serum diluited 1:100 in incubation buffer (washing buffer + foetal calf serum 2%) are then added to each well.

The sera are incubated for 45 minutes at 37°C and the wells are washed four times with washing buffer. 100 myl of anti-IgG or anti-IgM mouse monoclonal antibody (direct method) labelled with horse radish peroxidase (prepared, for example, according to the method described in: Galfre, G., Milstein, C. e Wright B., Nature, 227, 131-133, 1979 and labeled with peroxidase for example, according to the method described in: Shan. S. Wong, Chemistry of protein conjugation and cross-linking, CRC Press Inc, Boca Raton, Florida, USA, 253-254, 1993) diluted in incubation buffer at 37°C are then added; the wells are washed four times with washing buffer and 100 myl of substrate for peroxidase are added. After 15 minutes at room temperature the reaction is stopped by the addition of sulphuric acid and the developed colour is measured in a micro plate reader at a wavelenght of 450 nm.

Absorbance values achieved in Elisa with recombinant protein, purified Toscana virus (produced in cell cultures) and with the nucleo capside extracted therefrom, with sera from patients having a diagnosis of meningitis from Toscana virus (with PCR on liquor positive) (sera 1-14) and on two control sera from patients with meningitis but negative to PCR for Toscana virus (sera 15-16) are reported in table 1.

**TABLE I**

| | RECOMBINANT NUCLEOPROTEIN | | PURIFIED VIRUS | | PURIFIED NUCLEOCAPSIDE | |
|---|---|---|---|---|---|---|
| SERUM | IgG | IgM | IgG | IgM | IgG | IgM |
| 1 | 3.041 | 2.997 | 3.023 | 2.694 | 2.538 | 2.501 |
| 2 | 3.271 | 3.189 | 3.387 | 3.625 | 2.962 | 3.008 |
| 3 | 138 | 1.064 | 76 | 607 | 56 | 425 |
| 4 | 737 | 1.521 | 616 | 997 | 346 | 789 |
| 5 | 3.291 | 3.336 | 3.234 | 2.872 | 2.160 | 2.250 |
| 6 | 444 | 3.073 | 883 | 2.292 | 669 | 1.832 |
| 7 | 3.029 | 3.107 | 3.388 | 3.273 | 2.852 | 2.972 |
| 8 | 3.495 | 3.460 | 3.370 | 3.275 | 3.272 | 3.408 |
| 9 | 1.442 | 2.097 | 1.884 | 1.464 | 836 | 949 |
| 10 | 1.330 | 2.226 | 1.723 | 1.871 | 851 | 1.279 |
| 11 | 2.667 | 3.668 | 2.950 | 3.739 | 2.604 | 3.056 |
| 12 | 2.444 | 3.363 | 3.235 | 3.505 | 3.319 | 3.148 |
| 13 | 2.837 | 3.081 | 1.779 | 2.625 | 979 | 1.938 |
| 14 | 1.008 | 2.336 | 2.975 | 2.025 | 2.596 | 1.355 |
| 15 | 210 | 99 | 92 | 86 | 164 | 88 |
| 16 | 293 | 123 | 56 | 120 | 50 | 123 |

### Example 4

Results obtained with recombinant nucleoprotein in the assay of IgMs with the direct method and the "capturing" method (to demonstrate the equivalence of the direct method compared to the "capturing" method used in Example 3).

In the "capturing" method for the detection of specific IgMs, human serum under investigation, diluted 1:100 in incubation buffer, is incubated for 45 minutes in wells of micro plates in which a mouse anti-human IgMs monoclonal antibody has been previously immobilized. After the incubation period, the wells are washed four times with washing buffer, then added to each well are 100 myl of a biotinylated recombinant nucleoprotein-avidine complex labelled with horse radish peroxidase prepared as follows:
recombinant nucleoprotein is dialyzed against 0.1M bicarbonate buffer, pH 8.7, N-hydroxysuccinimidobiotin is then added to the solution at a 1:1 weight ratio. The solution is incubated at room temperature for three hours and subsequently dialyzed to PBS. Biotilynated nucleoprotein is then mixed with peroxidase labeled avidine (Boehringer Mannheim) to form the biotilynated nucleoprotein-avidine complex labeled with peroxidase. After 45 minutes of incubation at 37°C, the wells are washed four times with washing buffer, and 100 myl of substrate for peroxidase are then added. After 15 minutes of incubation at room temperature, the reaction is stopped by addition of sulphuric acid and the developed colour is measured in a micro plate reader at a wavelength of 450 nm.

**TABLE 2**

| | | | | RECOMBINANT NUCLEOPROTEIN | | PURIFIED VIRUS | PURIFIED NUCLEO CAPSIDE |
|---|---|---|---|---|---|---|---|
| SAMPLE | PCR* | IF** | WB*** | CAPTURE | DIRECT | DIRECT | DIRECT |
| T1 | + | ± | + | 3.061 | 2.997 | 2.694 | 2.501 |
| T2 | - | - | - | 72 | 99 | 86 | 88 |
| T3 | - | - | + | 387 | 533 | 352 | 241 |
| T4 | + | + | + | 2.940 | 3.189 | 3.625 | 3.008 |
| T5 | - | - | - | 126 | 123 | 120 | 123 |
| T6 | + | ± | + | 454 | 1.064 | 607 | 425 |
| T7 | N.D. | + | + | 3.456 | 3.472 | 3.761 | 3.283 |
| T8 | + | - | + | 767 | 1.521 | 997 | 789 |
| T9 | N.D. | - | + | 889 | 959 | 945 | 579 |
| T10 | + | - | + | 2.682 | 3.336 | 2.872 | 2.250 |
| T11 | + | ± | + | 3.307 | 3.073 | 2.292 | 1.832 |
| T12 | + | ± | + | 2.799 | 3.107 | 3.273 | 2.972 |
| T13 | + | + | + | 3.751 | 3.460 | 3.275 | 3.408 |
| T14 | + | - | + | 1.895 | 2.097 | 1.464 | 949 |
| T15 | + | - | + | 1.731 | 2.226 | 1.871 | 1.279 |
| T16 | + | + | + | 3.117 | 3.668 | 3.739 | 3.056 |
| T17 | - | - | - | 83 | 121 | 107 | 118 |
| T18 | N.D. | - | + | 3.504 | 3.568 | 3.363 | 3.365 |
| T19 | - | - | + | 3343 | 2.508 | 2.721 | 2.477 |
| T20 | + | + | + | 3.126 | 3.363 | 3.505 | 3.148 |
| T21 | + | - | + | 2.292 | 3.081 | 2.625 | 1.938 |
| T22 | - | - | + | 2.218 | 1.739 | 2.494 | 1.829 |
| T23 | + | N.D | + | 2.590 | 2.336 | 2.025 | 1.355 |

| | | | | | | | |
|---|---|---|---|---|---|---|---|
| * Polymerase chain reaction | | | | | | | |
| ** Indirect immunofluorescence performed on cells infected with Toscana virus; | | | | | | | |
| *** Western blotting performed with virus produced in cell cultures. | | | | | | | |
| N.D. Not Determined | | | | | | | |

### Example 5

Results obtained with 22 pediatric sera collected in the winter season, all negative for both IgG and 1gM antibodies to TV, in confirmation of the assay specificity with recombinant nucleoprotein of the invention. Data relative to IgMs are particularly significant, as their presence is considered a sign of recent or under way infection, due to the manifestation of TV in the summer period.

The last three lines of table 3 report values relative to a sample negative for both IgGs and IgMs, positive for IgGs and positive for IgMs, respectively.

**TABLE 3**

| | IgG | | IgM | | |
|---|---|---|---|---|---|
| | RECOMBINANT NUCLEOPROTEIN | PURIFIED VIRUS | RECOMBINANT NUCLEOPROTEIN | | PURIFIED VIRUS |
| SAMPLE | | | CAPTURE | DIRECT | DIRECT |
| 15550 | 399 | 95 | 331 | 80 | 110 |
| 16118 | 346 | 90 | 287 | 290 | 264 |
| 15251 | 318 | 84 | 184 | 143 | 199 |
| 15949 | 198 | 97 | 118 | 272 | 243 |
| 15542 | 475 | 89 | 259 | 118 | 100 |
| 15668 | 319 | 81 | 158 | 180 | 176 |
| 15689 | 228 | 128 | 387 | 217 | 321 |
| 15151 | 172 | 77 | 186 | 120 | 143 |
| 16186 | 331 | 96 | 129 | 123 | 168 |
| 15413 | 213 | 89 | 115 | 102 | 130 |
| 15259 | 211 | 93 | 228 | 179 | 160 |
| 15070 | 414 | 129 | 154 | 124 | 259 |
| 15210 | 274 | 123 | 145 | 103 | 137 |
| 16052 | 244 | 81 | 91 | 70 | 83 |
| 15171 | 54 | 56 | 57 | 59 | 64 |
| 16107 | 267 | 52 | 71 | 169 | 95 |
| 16117 | 473 | 51 | 134 | 103 | 119 |
| 16109 | 306 | 92 | 138 | 182 | 192 |
| 14037 | 218 | 102 | 111 | 87 | 110 |
| 15699 | 193 | 65 | 126 | 91 | 111 |
| 15805 | 194 | 67 | 85 | 84 | 87 |
| 16106 | 123 | 70 | 81 | 69 | 91 |
| negat. sample IgG/IgM | 170 | 68 | 86 | 83 | 125 |
| posit. sample IgG | 3314 | 2557 | - | - | - |
| posit. sample IgM | - | - | 2275 | 2789 | 2977 |

## Claims

1. A recombinant nucleoprotein of Toscana virus having the SEQ ID No: 1 or comprising the SEQ ID No: 1 or a portion or a modification thereof, that are reactive with antibodies of patients infected with TV, the infection being under way or previous, said SEQ ID No: 1 or portion or modification being optionally preceded and/or followed by amino acid sequences that do not alter the antigenic properties and biological functions of the nucleoprotein and/or that are required for the expression, isolation and/or purification of said SEQ ID No: 1 or portion or modification.

2. A nucleoprotein according to claim 1, having the SEQ ID No: 1.

3. A nucleoprotein according to claim 1, whereby the SEQ ID No: 1 is immediately preceded by the SEQ ID No: 2.

4. A process for the production and purification of the nucleoprotein according to any of previous claims, comprising:
- transcription of RNA corresponding to nucleotides 1070-1831 of the S segment of TV RNA to cDNA, through reverse transcriptase;
- amplification of the resulting cDNA, by polymerase chain reaction (PCR), using specific primers containing at the 5' end the nucleotide sequence recognized by an appropriate restriction enzyme;
- digestion of the amplificated cDNA with said enzyme and embedding thereof into a suitable expression plasmid in the cloning site of said enzyme;
- transformation with the aforesaid recombinant plasmid in an appropriate prokaryotic or eukaryotic expression system, such as, for example, bacteria, e.g. Escherichia coli and Bacillus subtilis, yeasts, for example, Saccharomyces cerevisiae, mammal cells, for example CHO cells, and insect cells, e.g. SF9 cells.
- expression of said nucleoprotein in said expression system;
- recovery and purification of the recombinant protein and possibly removal of amino acids that are extraneous to the SEQ ID No: 1 or a portion or a modification thereof that are reactive with antibodies from TV-infected patients, where the infection is under way or previous, said SEQ ID No: 1 or portion or modification being optionally preceded and/or followed by amino acid sequences that do not alter the antigenic properties and biological functions of the nucleoprotein and/or that are required for the expression, isolation and/or purification of said SEQ ID No: 1 or portion or modification.

5. A proces according to claim 4, whereby the nucleoprotein has the SEQ ID No: 1, possibly immediately preceded by the SEQ ID No: 2, and in which the production of said nucleoprotein takes place by expression in Escherichia coli of cDNA of the gene coding for the nucleoprotein, located in S segment of viral RNA, comprising:
- transcription of RNA corresponding to nucleotides 1070-1831 of the S segment of TV RNA in cDNA, through reverse transcriptase;
- amplification of the resulting cDNA, by means of the polymerase chain reaction (PCR), using specific primers containing at the 5' end the nucleotide sequence recognized by an appropriate restriction enzyme;
- digestion of amplified cDNA by said enzyme which is then embedded into a suitable expression plasmid in the cloning site of said enzyme;
- transformation with the said recombinant plasmid of BL21 (ADE3) strain of E. coli;
- induction of expression of said nucleoprotein by addition to said bacterial culture of isopropyl β-thiogalactopiranoside;
- purification of said recombinant nucleoprotein by immobilized metal affinity chromatography and possible removal of amino acids of the SEQ ID No: 2 through digestion with thrombine.

6. A process according to claim 5, in which the primers are a sense primer having the SEQ ID No: 3 and an antisense primer having the SEQ ID No: 4.

7. A process according to anyone of claims 4-6, in which the purification of the recombinant protein comprises:
- extraction of the recombinant protein from bacteria by sonication;
- supernatant chromatography on a chromatographic column containing a resin with immobilized metal, preferably nickel, such as Ni-NTA resin.

8. A method for detecting anti Toscana virus antibodies, comprising the use of recombinant nucleoprotein according to any of claims 1-3.

9. A method according to claim 8, comprising an immunoenzymatic method to detect antibodies of the IgG and IgM classes.

10. A method according to claim 9, whereby the nucleoprotein is immobilized on a solid phase.

11. A method according to claim 9 or 10, whereby specific antibodies present in the sample under investigation are detected by the addition of a anti-human IgGs or anti-human IgMs monoclonal antibody that is labeled with peroxidase.

12. A method according to any of claims 9-11, whereby, for detecting specific IgMs, these are captured by a anti-human IgMs monoclonal antiboby immobilized on solid phase, and then detected by the addition of a biotinylated recombinant nucleoprotein- avidine complex labelled with peroxidase.

13. A diagnostic kit including the recombinant nucleoprotein according to anyone of claims 1-3.
